(19) Europäisches Patentamt
European Patent Office
Office européen des brevets

(11) **EP 2 659 984 B1**

(12) **EUROPEAN PATENT SPECIFICATION**

(45) Date of publication and mention
of the grant of the patent:
**14.11.2018 Bulletin 2018/46**

(51) Int Cl.:
**B03C 1/28** (2006.01)     **G01N 33/483** (2006.01)

(21) Application number: **12176073.0**

(22) Date of filing: **12.07.2012**

(54) **Apparatus for self-extracting cells using magnetic force and method for self-extracting cells using the same**

Vorrichtung zur Selbstextraktion von Zellen mit Magnetkraft und Verfahren zur Selbstextraktion von Zellen damit

Appareil d'extraction automatique de cellules utilisant une force magnétique et procédé d'extraction automatique de cellules l'utilisant

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2012 KR 20120045751**

(43) Date of publication of application:
**06.11.2013 Bulletin 2013/45**

(73) Proprietor: **Korea Institute of Machinery & Materials**
**Daejeon 305-343 (KR)**

(72) Inventors:
• **Chang, Sung Hwan**
**Daejeon 305-343 (KR)**
• **Yoo, Yeong-Eun**
**Daejeon 305-343 (KR)**
• **Kim, Jung Yup**
**Daejeon 305-343 (KR)**
• **Hyun, Seung Min**
**Daejeon 305-343 (KR)**
• **Whang, Kyung-Hyun**
**Daejeon 305-343 (KR)**

(74) Representative: **Delorme, Nicolas et al**
**Cabinet Germain & Maureau**
**BP 6153**
**69466 Lyon Cedex 06 (FR)**

(56) References cited:
**EP-A1- 0 637 999**     **JP-A- 2009 109 232**
**US-A1- 2009 188 864**

**Description**

**BACKGROUND OF THE INVENTION**

**(a) Field of the Invention**

The present invention relates to an apparatus for self-extracting a cell using a magnetic field, and a method using the same, and more particularly, to an apparatus for self-extracting a cell using a magnetic field to easily extract an effective cell from a cell solution, and a method using the same.

**(b) Description of the Related Art**

Generally, since a biochemical sample exists while two kinds or more materials are mixed with each other, a separation technology for analyzing only a desired component or purifying only a predetermined component in a mixture is very important during a pretreatment process of the sample. Particularly, a preparation process of the sample such as purification and separation is an essential technology to be performed prior to a subsequent analysis process even in a lab-on-a-chip that is a concept of treating a small amount of sample at a high speed and high efficiency by integrating a fine flow path, a mixer, a pump, a valve and the like on a single chip.

Further, cell-based diagnostics that are important in a biological or medical analysis are formed of blood analyses, cell studies, microorganism analyses and tissue transplant. Recently, unification and integration of processes of the cell-based diagnostics into a microfluidic device form have been studied in accordance with development of cell studies, cell analyses, and protein and DNA analysis technologies.

However, in the case of a known cell separation method and apparatus using a fine fluid channel and the like, cell separation performance is unsatisfactory, and accordingly, it is difficult to substantially use the method and apparatus.

It is known from document US2009/188864 a microfiltration apparatus and method for separating cells, such as circulating tumor cells, from a sample using a microfiltration device having a top porous membrane and a bottom porous membrane. The porous membranes are formed from parylene and assembled using microfabrication techniques. The porous membranes are arranged so that the pores in the top membrane are offset from the pores in the bottom membrane.

The above information disclosed in this Background section is only for enhancement of understanding of the background of the invention and therefore it may contain information that does not form the prior art that is already known in this country to a person of ordinary skill in the art.

**SUMMARY OF THE INVENTION**

The present invention has been made in an effort to provide an apparatus for self-extracting a cell using a magnetic field capable of selectively separating the cell using a separation channel and actively preventing an effective cell from blocking the separation channel by generating the magnetic field, and a method using the same.

An exemplary embodiment of the present invention provides an apparatus for self-extracting a cell using a magnetic field to extract an effective cell that is a separation target from a cell solution, including: a flow path casing including an upper substrate and a lower substrate having a magnetic property combined with each other, and a fluid path formed to fluidize the cell solution therein; a separation portion disposed on the fluid path and provided with a separation channel selectively blocking the effective cell that is an extraction target in the cell included in the cell solution from passing therethrough; and a magnetic field control portion forming the magnetic field in the flow path portion to separate the effective cell blocking the separation channel from the separation channel.

The separation channel may be constituted so that a width thereof becomes gradually narrow in a fluid direction of the cell solution.

The separation portion may include a plurality of fine structures spaced apart from each other so that the width thereof becomes gradually wide in the fluid direction of the cell solution to form the separation channel.

The plurality of fine structures may be constituted so that a gradient of the magnetic field generated between the magnetic field control portion and the lower substrate is increased.

An addition port for adding the cell solution and a discharge port for discharging a non-effective cell passing through the separation portion to the outside may be formed at both ends of the fluid path.

The lower substrate may be formed by curing a mixed solution of a polymer resin and a ferromagnetic particle.

The magnetic field control portion may operate the magnetic field in the fluid path to perform controlling in the case where the separation channel is blocked by the effective cell.

The magnetic field control portion may be constituted by an electromagnet to control an applied current, thereby controlling the intensity of the magnetic field.

Another exemplary embodiment of the present invention provides a method for extracting a cell using a magnetic field, including: an addition step of adding a cell solution including an effective cell that is an extraction target and a non-effective cell that is a non-extraction target to a fluid path of a flow path casing; an extraction step of passing the cell solution through each of a plurality of separation channels to pass the non-effective cell therethrough and selectively extract the effective cell; and a magnetic field generation step of generating the magnetic field in the fluid path so as not to block the separation channel by the effective cell.

In the magnetic field generation step, the magnetic field may be generated while the addition step is performed to basically prevent the effective cell from blocking the separation channel.

In the magnetic field generation step, the magnetic field may be selectively generated to separate the effective cell blocking the separation channel from the separation channel.

According to the exemplary embodiments of the present invention, there is provided an apparatus for self-extracting a cell using a magnetic field to easily self-extract an effective cell that is a separation target using a separation channel magnetic field.

Further, it is possible to basically prevent the effective cell that is the extraction target from blocking the separation channel by generating the magnetic field.

In addition, it is possible to self-remove the effective cell by selectively generating the magnetic field only in the case where the separation channel is blocked by the effective cell.

## BRIEF DESCRIPTION OF THE DRAWINGS

FIG. 1 is a schematic perspective view of an apparatus for self-extracting a cell using a magnetic field according to an exemplary embodiment of the present invention.

FIG. 2 is a schematic exploded perspective view of the apparatus for self-extracting the cell using the magnetic field of FIG. 1.

FIG. 3 is a schematic top plan view of the apparatus for self-extracting the cell using the magnetic field of FIG. 1, from which an upper substrate is removed.

FIG. 4 illustrates a cross-section of the apparatus for self-extracting the cell using the magnetic field of FIG. 1, which is taken along the cut line IV - IV'.

FIGS. 5 and 6 illustrate an operation principle of separating an effective cell by the apparatus for self-extracting the cell using the magnetic field of FIG. 1.

FIG. 7 illustrates a principle of separating the effective cell blocking a separation channel in the apparatus for self-extracting the cell using the magnetic field of FIG. 1.

FIG. 8 illustrates a cross-section of the apparatus for self-extracting the cell using the magnetic field of FIG. 7, which is taken along the cut line VIII-VIII'.

## DETAILED DESCRIPTION OF THE EMBODIMENTS

Hereinafter, an apparatus for self-extracting a cell using a magnetic field according to an exemplary embodiment of the present invention will be described in detail with reference to the accompanying drawings.

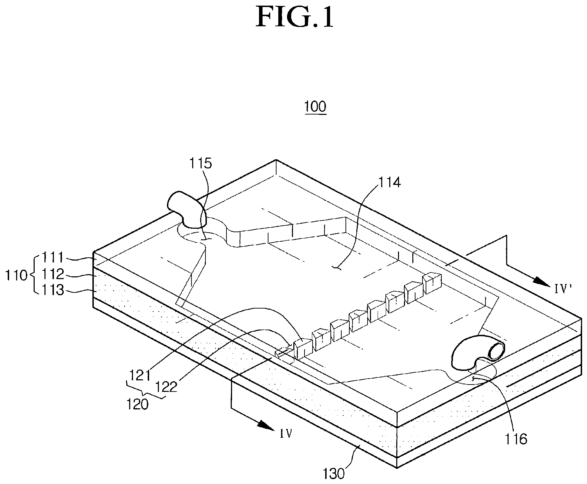

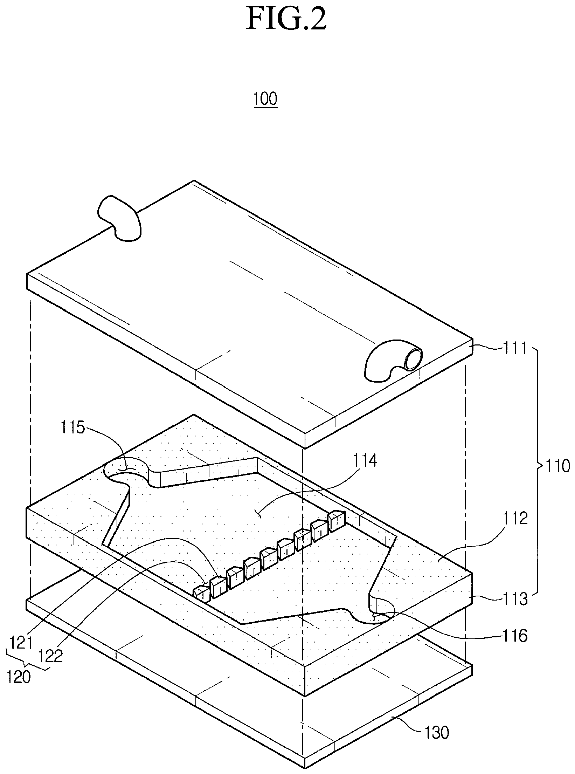

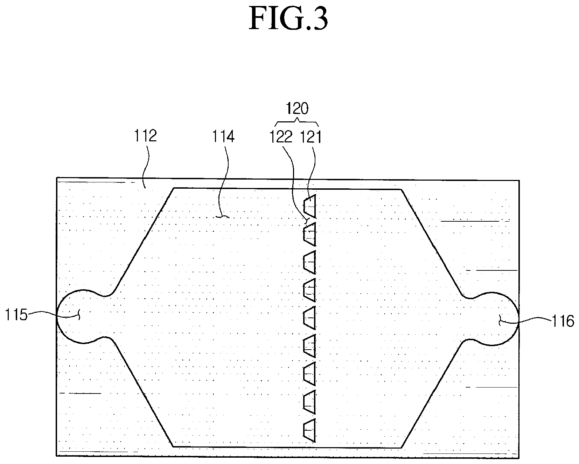

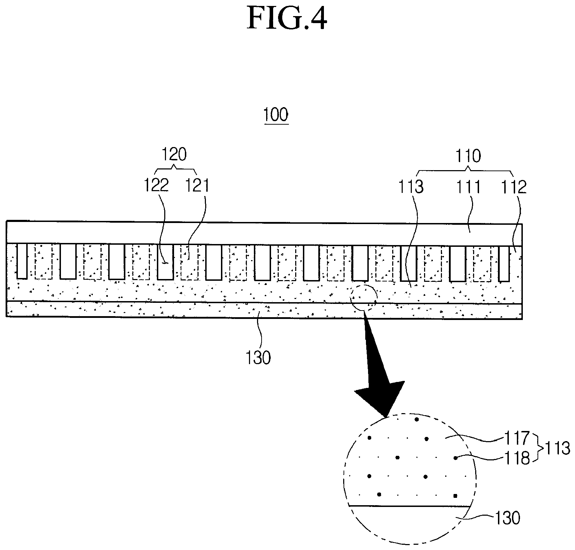

FIG. 1 is a schematic perspective view of an apparatus for self-extracting a cell using a magnetic field according to an exemplary embodiment of the present invention, FIG. 2 is a schematic exploded perspective view of the apparatus for self-extracting the cell using the magnetic field of FIG. 1, FIG. 3 is a schematic top plan view of the apparatus for self-extracting the cell using the magnetic field of FIG. 1, from which an upper substrate is removed, and FIG. 4 illustrates a cross-section of the apparatus for self-extracting the cell using the magnetic field of FIG. 1, which is taken along the cut line IV - IV'.

Referring to FIGS. 1 to 4, an apparatus 100 for self-extracting a cell using a magnetic field according to the exemplary embodiment of the present invention relates to an apparatus for extracting a cell which selectively self-extracts only an effective cell 20 from a cell solution including the effective cell 20 that is an extraction target and a non-effective cell 10 that is a non-extraction target, and includes a flow path casing 110, a separation portion 120 and a magnetic field control portion 130.

A fluid path 114 for fluidizing the cell solution is formed in the flow path casing 110, and the flow path casing 110 includes an upper substrate 111, a side wall 112 and a lower substrate 113.

The upper substrate 111 is combined with the side wall 112 and the lower substrate 113 to be described below so as to form the fluid path 114, and has a flat plate shape. In the present exemplary embodiment, polydimethylsiloxane (PDMS), polytetrafluoroethylene (PTFE), polymethyl methacrylate (PMMA), cycloolefin copolymer (COC) and the like may be used as the upper substrate 111, but any general polymer material may be used without limitation.

The side wall 112 is interposed between the upper substrate 111 and the lower substrate 113 to be described below to connect both constituent elements to each other, and forms a space in the flow path casing 110 so as to provide the fluid path 114, an addition port 115 and a discharge port 116 therein.

That is, the side wall 112 constitutes a border between the upper substrate 111 and the lower substrate 113, and forms a space as the fluid path 114 therein. Specifically, the fluid path 114 where a central region has the largest width and the width becomes narrow as going to both ends is formed in the side wall 112. Further, the addition port 115 for adding the cell solution is formed at one end of the fluid path 114, and the discharge port 116 for discharging the non-effective cell 10 passing through the separation portion 120 to the outside is formed at the other end of the fluid path 114.

Meanwhile, the lower substrate 113 to be described below and the side wall 112 may be integrally formed of the same material in a single process, but may be separately manufactured through precision processing to be firmly attached to each other.

The lower substrate 113 is integrally manufactured with the side wall 112 described above and the separation portion 120 to be described below to finish the fluid path 114 at the lower part thereof, and is formed to have the same flat plate shape as the upper substrate 111.

The separation portion 120 is provided on the fluid path 114 in the flow path casing 110 to act as a filter for selectively extracting only the effective cell 20 that is the extraction target from the cell solution and act as a structure for increasing a gradient of the magnetic field generated from the magnetic field control portion 130 to be described below, and is constituted by a plurality of fine structures 121 and a separation channel 122 between the fine structures 121.

The plurality of fine structures 121 are spaced apart from each other in a width direction of the fluid path 114 so as to form the separation channel 122, and each fine structure 121 has a shape where a width becomes gradually wide in a fluid direction (D) of the cell solution.

Accordingly, the separation channel 122 formed in a space between the fine structures 121 where the width becomes gradually wide in the fluid direction (D) of the cell solution has a shape where a width becomes gradually narrow in the fluid direction (D) of the cell solution on the contrary to the shape of the fine structure 121.

Meanwhile, the width of the fine structure 121, the degree of change in width, the height and the like needs to be designed in overall consideration of a fluid speed of the cell solution, a kind of the cell solution, and a kind and a size of the effective cell 20 to be extracted.

Further, the fine structure 121 acts as a structure for increasing a gradient of the magnetic field generated between the magnetic field control portion 130 to be described below and the fine structure and it is necessary to determine a shape in consideration of the gradient of the magnetic field to be generated.

Meanwhile, the side wall 112, the lower substrate 113 and the separation portion 120 that are integrally formed are manufactured by curing a mixing material where ferromagnetic particles 118 are mixed with a liquid type polymer resin 117 so as to have a magnetic property.

Nano or micro particles of nickel (Ni), cobalt (Co), iron (Fe) and the like may be used as the ferromagnetic particles 118 used in the present exemplary embodiment. Further, in the exemplary embodiment, polydimethylsiloxane (PDMS), polytetrafluroethylene (PTFE), polymethyl methacrylate (PMMA), cycloolefin copolymer (COC) and the like may be used as the polymer resin 117, but any general polymer material may be used without limitation.

In addition, a separate non-ferromagnetic additive may be further included in the mixing material to improve characteristics such as strength, electric conductivity and thermal conductivity.

Carbon nanotubes (CNT), carbon fibers, glass fibers or two or more thereof may be mixed and used while being mixed with each other as the non-ferromagnetic additive, and the non-ferromagnetic additive is not limited thereto as long as the non-ferromagnetic additive helps to improve the characteristics of the mixing material after curing.

Therefore, according to the structure of the flow path casing 110 and the separation portion 120 described above, the fluid path where the addition port 115 and the discharge port 116 are formed at both ends is provided in the flow path casing, and since the lower substrate 113, the side wall 112 and the separation portion 120 include the uniformly distributed ferromagnetic particles 118, the magnetic property is ensured.

The magnetic field control portion 130 is operated together with the lower substrate 113 and the ferromagnetic particles 118 in the fine structure 121 to generate the magnetic field in order to basically prevent the effective cell 20 that is the extraction target from blocking the separation channel 122 or selectively be operated only in the case where the effective cell 20 blocks the separation channel to prevent the blocking, and is provided at the lower part of the lower substrate 113.

Further, in the exemplary embodiment, the magnetic field control portion 130 may be provided in an electromagnet form to control the intensity of magnetic property and operation, such that the intensity of total magnetic field may be controlled by controlling a quantity of applied current, but the form of the magnetic field control portion 130 is not limited thereto and the magnetic field control portion 130 may be provided in a permanent magnet form.

An electromagnet may be provided, but a matter such as the permanent magnet which can generate the magnetic field may be used without limitation.

Hereinafter, operation of the apparatus 100 for self-extracting the cell using the magnetic field according to an exemplary embodiment will be described.

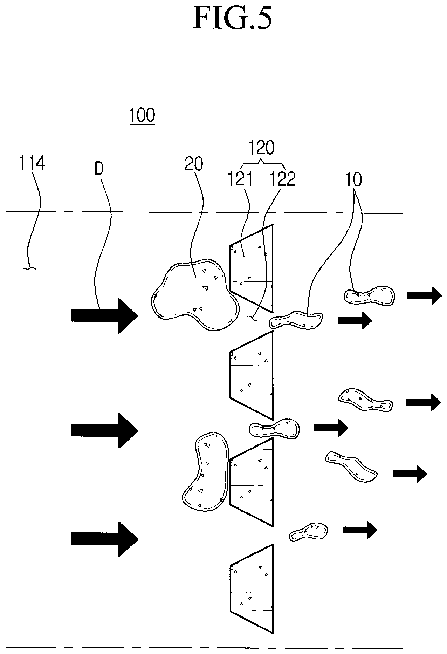

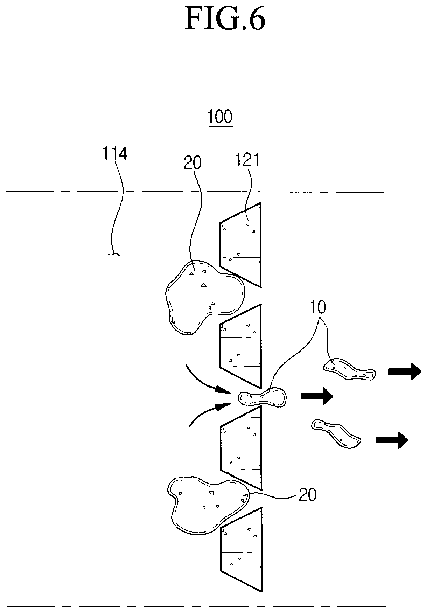

FIGS. 5 and 6 illustrate an operation principle of separating an effective cell by the apparatus for self-extracting the cell using the magnetic field of FIG. 1.

First, the cell solution including the effective cell 20 that is the extraction target and the non-effective cell 10 that is not the extraction target is continuously added through the addition port 115 at the end of the fluid path 114. The cell solution added through the addition port 115 is continuously fluidized along the fluid path 114 and reaches the separation portion 120.

In this case, as shown in FIG. 5, the non-effective cell 10 having a diameter that is smaller than the width of the separation channel 122 passes through the separation channel 122 of the separation portion 120 and is discharged through the discharge port 116 at the end of the fluid path 114 to the outside.

Furthermore, as shown in FIG. 6, the effective cell having a diameter that is larger than the width of the separation channel 122 does not completely pass through the separation channel 122 but is extracted.

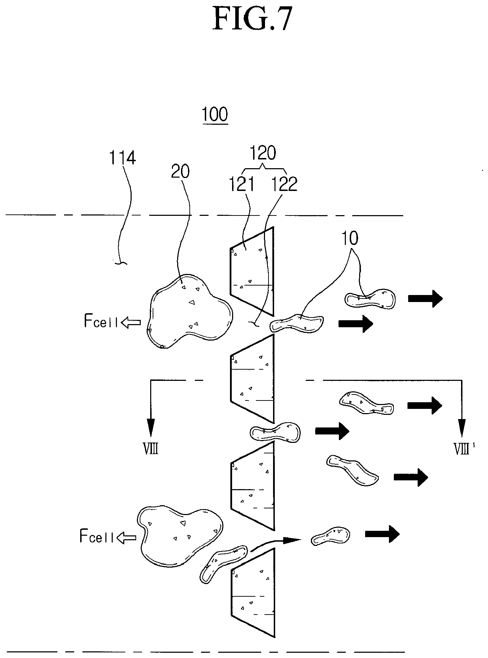

FIG. 7 illustrates a principle of separating the effective cell blocking a separation channel in the apparatus for self-extracting the cell using the magnetic field of FIG. 1.

Meanwhile, the magnetic field control portion 130 generates the magnetic field between the lower substrate 113 and the fine structure 121 while the cell solution is fluidized as described above, and the generated magnetic field is formed in the fluid path 114.

As shown in FIG. 7, the effective cell 20 may not block the separation channel 122 but be self-extracted by force ($F_{cell}$) applied to the effective cell 20 from the magnetic field generated by the operation of the magnetic field control portion 130.

(Equation 1)

$$F_{cell} = \frac{1}{2} \frac{\triangle \chi \cdot V_{cell}}{\mu_0} \nabla |B|^2$$

$$F_{cell} = \frac{1}{2} \frac{\triangle \chi \cdot V_{cell}}{\mu_0} \overline{\nabla} |B|^2$$

($F_{cell}$: force applied to the effective cell, $V_{cell}$: volume of the effective cell, $\nabla|B|$: gradient of the magnetic field, $\triangle_\chi$: difference in magnetic susceptibility of the effective cell and the cell solution, and $\mu_0$: magnetic permeability in a vacuum)

That is, turning to the aforementioned self-extracting of the effective cell 20, the force ($F_{cell}$) applied to the effective cell 20 by the magnetic field generated from the magnetic field control portion 130 with the exception of gravity and fluidizing force by the cell solution may be represented by Equation 1.

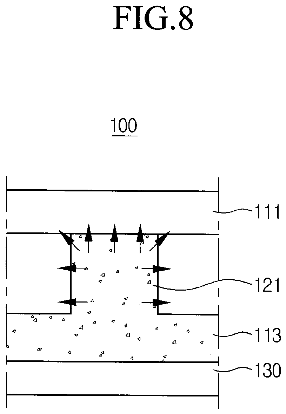

FIG. 8 illustrates a cross-section of the apparatus for self-extracting the cell using the magnetic field of FIG. 7, which is taken along the cut line VIII-VIII'.

In this case, as shown in FIG. 8, since the fine structure 121 having a shape protruding from the lower substrate 113 to the upper side functions to increase the gradient ($\nabla|B|$) of the magnetic field in the fluid path 114, and as in Equation 1, the force ($F_{cell}$) directly applied to the effective cell 20 in order to separate the effective cell 20 from the separation channel 122 is in proportion to the square of the gradient ($\nabla|B|$) of the magnetic field, the fine structure 121 increases force used to remove cell separation from the separation channel 122 to basically prevent the effective cell 20 from blocking the separation channel 122, thereby improving an entire self-extracting ability of the effective cell 20.

Meanwhile, in the present exemplary embodiment, the effective cell 20 is basically prevented from blocking the separation channel 122 by operating the magnetic field control portion 130 during the extraction process of the cell solution, such that the effective cell 20 is self-extracted, but in another modified embodiment, the effective cell 20 blocking the separation channel 122 may be extracted from the separation channel 122 by selectively generating the magnetic field by the magnetic field control portion 130 only in the case where the effective cell 20 blocks the separation channel 122.

Therefore, according to the present invention, it is possible to basically prevent the effective cell from blocking the separation channel and easily self-extract the effective cell by generating the magnetic field during the extraction operation or selectively generating the magnetic field.

The scope of the present invention is not limited to the aforementioned exemplary embodiments, but may be implemented by various exemplary embodiments within the range of the accompanying claims. Although the preferred embodiments of the present invention have been disclosed for illustrative purposes, those skilled in the art will appreciate that various modifications, additions and substitutions are possible, without departing from the scope of the invention as disclosed in the accompanying claims. Accordingly, such modifications, additions and substitutions should also be understood to fall within the scope of the present invention.

**<Description of symbols>**

100: Apparatus for self-extracting a cell using a magnetic field according to an exemplary embodiment of the present invention
110: Flow path casing 120: Separation portion
130: Magnetic field control portion

**Claims**

1. An apparatus (100) for self-extracting a cell to extract an effective cell (20) that is a separation target from a cell solution,
comprising:

   a flow path casing (110) including an upper substrate (111) and a lower substrate (113) combined with each other, and a fluid path (114) formed to fluidize the cell solution therein;
   a separation portion (120) disposed on the fluid path (114) and provided with a separation channel (122) selectively blocking the effective cell (20) that is an extraction target in the cell included in the cell solution from passing therethrough; and **characterized in that**
   the upper substrate (111) and the lower substrate (113) have a magnetic property; and **in that** the apparatus (100) further comprises a magnetic field control portion (130) forming a magnetic field in the flow path portion to separate the effective cell (20) blocking the separation channel (122) from the separation channel (122).

2. The apparatus (100) for self-extracting a cell of claim 1, wherein:
the separation channel (122) is constituted so that a width thereof becomes gradually narrow in a fluid direction of the cell solution.

3. The apparatus (100) for self-extracting a cell of claim 2, wherein:
the separation portion (120) includes a plurality of fine structures (121) spaced apart from each other so that the width thereof becomes gradually wide in the fluid direction of the cell solution to form the separation channel (122).

4. The apparatus (100) for self-extracting a cell of claim 3, wherein:
the plurality of fine structures (121) are constituted so that a gradient of the magnetic field generated between the magnetic field control portion (130) and the lower substrate (113) is increased.

5. The apparatus (100) for self-extracting a cell of claim 4, wherein:
an addition port (115) for adding the cell solution and a discharge port (116) for discharging a non-effective cell (20) passing through the separation portion (120) to the outside are formed at both ends of the fluid path (114).

6. The apparatus (100) for self-extracting a cell of claim 1, wherein:
the lower substrate (113) is formed by curing a mixed solution of a polymer resin and a ferromagnetic particle.

7. The apparatus (100) for self-extracting a cell of claim 1, wherein:
the magnetic field control portion (130) operates the magnetic field in the fluid path (114) to perform controlling in the case where the separation channel (122) is blocked by the effective cell (20).

8. The apparatus (100) for self-extracting a cell of claim 1, wherein:
the magnetic field control portion (130) is constituted by an electromagnet to control an applied current, thereby controlling the intensity of magnetic field.

9. A method for extracting a cell by using the apparatus (100) for self-extracting the cell of claim 1, comprising:

an addition step of adding a cell solution including an effective cell (20) that is an extraction target and a non-effective cell (20) that is a non-extraction target to the fluid path (114) of the flow path casing;
an extraction step of passing the cell solution through each of a plurality of separation channel (122)s to pass the non-effective cell (20) therethrough and selectively extract the effective cell (20); and
a magnetic field generation step of generating the magnetic field in the fluid path (114) so as not to block the separation channel (122) by the effective cell (20).

10. The method for extracting a cell of claim 9, wherein:
in the magnetic field generation step, the magnetic field is generated while the addition step is performed to basically prevent the effective cell (20) from blocking the separation channel (122).

11. The method for extracting a cell of claim 9, wherein:
in the magnetic field generation step, the magnetic field is selectively generated to separate the effective cell (20) blocking the separation channel (122) from the separation channel (122).

**Patentansprüche**

1. Vorrichtung (100) zur Selbstextraktion einer Zelle, um eine effektive Zelle (20), die ein Trennziel ist, aus einer Zelllösung zu extrahieren,
umfassend:

ein Strömungspfadgehäuse (110), das ein oberes Substrat (111) und ein unteres Substrat (113), die miteinander kombiniert sind, und einen Fluidpfad (114) einschließt, der dazu gebildet ist, die Zelllösung in demselben zu fluidisieren;
einen Trennabschnitt (120), der im Fluidpfad (114) angeordnet und mit einem Trennkanal (122) versehen ist, welcher selektiv die effektive Zelle (20), die ein Extraktionsziel ist, in der in der Zelllösung eingeschlossenen Zelle dagegen sperrt, durch denselben hindurchzutreten; und **dadurch gekennzeichnet, dass**
das obere Substrat (111) und das untere Substrat (113) eine magnetische Eigenschaft aufweisen; und dadurch, dass
die Vorrichtung (100) weiter einen Magnetfeldsteuerabschnitt (130) umfasst, der ein Magnetfeld im Strömungspfadabschnitt bildet, um die effektive Zelle (20), die den Trennkanal (122) versperrt, vom Trennkanal (122) zu trennen.

2. Vorrichtung (100) zur Selbstextraktion einer Zelle nach Anspruch 1, wobei:
der Trennkanal (122) so aufgebaut ist, dass eine Breite desselben in einer Fluidrichtung der Zelllösung zunehmend schmal wird.

3. Vorrichtung (100) zur Selbstextraktion einer Zelle nach Anspruch 2, wobei:
der Trennabschnitt (120) eine Vielzahl von feinen Strukturen (121) einschließt, die so voneinander beabstandet sind, dass die Breite derselben in der Fluidrichtung der Zelllösung zunehmend breit wird, um den Trennkanal (122) zu bilden.

4. Vorrichtung (100) zur Selbstextraktion einer Zelle nach Anspruch 3, wobei:
die Vielzahl von feinen Strukturen (121) so aufgebaut sind, dass ein Gradient des erzeugten Magnetfelds zwischen dem Magnetfeldsteuerabschnitt (130) und dem unteren Substrat (113) erhöht wird.

5. Vorrichtung (100) zur Selbstextraktion einer Zelle nach Anspruch 4, wobei:
an beiden Enden des Fluidpfads (114) eine Eintragsöffnung (115) zum Eintragen der Zelllösung, und eine Austragsöffnung (116) zum Austragen einer durch den Trennabschnitt (120) hindurchtretenden nicht effektiven Zelle (10) zur Außenseite gebildet sind.

6. Vorrichtung (100) zur Selbstextraktion einer Zelle nach Anspruch 1, wobei:
das untere Substrat (113) durch Aushärten einer Mischlösung aus einem Polymerharz und einem ferromagnetischen Teilchen gebildet wird.

**7.** Vorrichtung (100) zur Selbstextraktion einer Zelle nach Anspruch 1, wobei:
der Magnetfeldsteuerabschnitt (130) das Magnetfeld im Fluidpfad (114) so betreibt, dass Steuerung in dem Fall durchgeführt wird, in dem der Trennkanal (122) von der effektiven Zelle (20) versperrt wird.

**8.** Vorrichtung (100) zur Selbstextraktion einer Zelle nach Anspruch 1, wobei:
der Magnetfeldsteuerabschnitt (130) aus einem Elektromagneten aufgebaut ist, um einen angelegten Strom zu steuern, wodurch die Magnetfeldstärke gesteuert wird.

**9.** Verfahren zum Extrahieren einer Zelle durch Verwenden der Vorrichtung (100) zur Selbstextraktion der Zelle nach Anspruch 1, umfassend:

einen Eintragsschritt des Eintragens einer Zelllösung, die eine effektive Zelle (20), welche ein Extraktionsziel ist, und eine nicht effektive Zelle (10) einschließt, welche kein Extraktionsziel ist, in den Fluidpfad (114) des Ström ungspfadgehäuses;
einen Extraktionsschritt des Hindurchleitens der Zelllösung durch jeden aus einer Vielzahl von Trennkanälen (122), um die nicht effektive Zelle (10) durch dieselben hindurchzuleiten und selektiv die effektive Zelle (20) zu extrahieren; und
einen Magnetfelderzeugungsschritt des Erzeugens des Magnetfelds im Fluidpfad (114) so, dass der Trennkanal (122) nicht von der effektiven Zelle (20) versperrt wird.

**10.** Verfahren zum Extrahieren einer Zelle nach Anspruch 9, wobei:
im Magnetfelderzeugungsschritt das Magnetfeld erzeugt wird, während der Eintragsschritt durchgeführt wird, um grundsätzlich die effektive Zelle (20) daran zu hindern, den Trennkanal (122) zu versperren.

**11.** Verfahren zum Extrahieren einer Zelle nach Anspruch 9, wobei:
im Magnetfelderzeugungsschritt das Magnetfeld selektiv erzeugt wird, um die effektive Zelle (20), die den Trennkanal (122) versperrt, vom Trennkanal (122) zu trennen.

**Revendications**

**1.** Appareil (100) pour auto-extraction d'une cellule pour extraire une cellule efficace (20) qui est une cible de séparation d'une solution cellulaire,
comprenant :

un boîtier de trajet d'écoulement (110) comportant un substrat supérieur (111) et un substrat inférieur (113) combinés entre eux, et un trajet de fluide (114) formé pour fluidifier la solution cellulaire dans celui-ci ;
une partie de séparation (120) disposée sur le trajet de fluide (114) et pourvue d'un canal de séparation (122) empêchant sélectivement la cellule efficace (20) qui est une cible d'extraction dans la cellule incluse dans la solution cellulaire de passer à travers celui-ci ; et **caractérisé en ce que**
le substrat supérieur (111) et le substrat inférieur (113) ont une propriété magnétique ; et **en ce que**
l'appareil (100) comprend en outre une partie de commande de champ magnétique (130) formant un champ magnétique dans la partie de trajet d'écoulement pour séparer la cellule efficace (20) bloquant le canal de séparation (122) du canal de séparation (122).

**2.** Appareil (100) pour auto-extraction d'une cellule de la revendication 1, dans lequel :
le canal de séparation (122) est constitué de sorte que la largeur de celui-ci devienne progressivement étroite dans une direction de fluide de la solution cellulaire.

**3.** Appareil (100) pour auto-extraction d'une cellule de la revendication 2, dans lequel :
la partie de séparation (120) comporte une pluralité de structures fines (121) espacées les unes des autres de sorte que la largeur de celles-ci devienne progressivement grande dans la direction de fluide de la solution cellulaire pour former le canal de séparation (122).

**4.** Appareil (100) pour auto-extraction d'une cellule de la revendication 3, dans lequel :
la pluralité de structures fines (121) est constituée de sorte qu'un gradient du champ magnétique généré entre la partie de commande de champ magnétique (130) et le substrat inférieur (113) augmente.

**5.** Appareil (100) pour auto-extraction d'une cellule de la revendication 4, dans lequel :
un orifice d'ajout (115) pour ajouter la solution cellulaire et un orifice d'évacuation (116) pour évacuer une cellule non efficace (10) passant à travers la partie de séparation (120) vers l'extérieur sont formés aux deux extrémités du trajet de fluide (114).

**6.** Appareil (100) pour auto-extraction d'une cellule de la revendication 1, dans lequel :
le substrat inférieur (113) est formé par durcissement d'une solution mixte d'une résine polymère et d'une particule ferromagnétique.

**7.** Appareil (100) pour auto-extraction d'une cellule de la revendication 1, dans lequel :
la partie de commande de champ magnétique (130) active le champ magnétique dans le trajet de fluide (114) pour effectuer la commande dans le cas où le canal de séparation (122) est bloqué par la cellule efficace (20).

**8.** Appareil (100) pour auto-extraction d'une cellule de la revendication 1, dans lequel :
la partie de commande de champ magnétique (130) est constituée par un électroaimant pour commander un courant appliqué, commandant ainsi l'intensité du champ magnétique.

**9.** Procédé d'extraction d'une cellule en utilisant l'appareil (100) pour auto-extraction de la cellule de la revendication 1, comprenant :

une étape d'ajout qui consiste à ajouter une solution cellulaire comportant une cellule efficace (20) qui est une cible d'extraction et une cellule non efficace (10) qui n'est pas une cible d'extraction au trajet de fluide (114) du boîtier de trajet d'écoulement ;
une étape d'extraction qui consiste à faire passer la solution cellulaire à travers chacun d'une pluralité de canaux de séparation (122) pour faire passer la cellule non efficace (10) à travers ceux-ci et extraire sélectivement la cellule efficace (20) ; et
une étape de génération de champ magnétique qui consiste à générer le champ magnétique dans le trajet de fluide (114) afin de ne pas bloquer le canal de séparation (122) par la cellule efficace (20).

**10.** Procédé d'extraction d'une cellule de la revendication 9, dans lequel :
dans l'étape de génération de champ magnétique, le champ magnétique est généré pendant que l'étape d'ajout est effectuée pour empêcher essentiellement la cellule efficace (20) de bloquer le canal de séparation (122).

**11.** Procédé d'extraction d'une cellule de la revendication 9, dans lequel :
dans l'étape de génération de champ magnétique, le champ magnétique est sélectivement généré pour séparer la cellule efficace (20) bloquant le canal de séparation (122) du canal de séparation (122).

# FIG.1

100

# FIG.2

100

# FIG.3

# FIG.4

# FIG.5

# FIG.6

# FIG.7

# FIG.8

**EP 2 659 984 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2009188864 A